# EUROPEAN PATENT APPLICATION

(11) **EP 4 710 850 A1**
(43) Date of publication of application: **18.03.2026**
(21) Application number: 24803348.2
(22) Date of filing: 19.04.2024
(51) Int. Cl.: A61B 5/055, F16L 11/12, F17D 1/08

(54) **TRANSMISSION DEVICE AND TRANSMISSION METHOD**

(30) Priority: 09.05.2023 JP 2023077347
(71) Applicant: NATIONAL INSTITUTES FOR QUANTUM SCIENCE AND TECHNOLOGY, Chiba-shi, Chiba 263-8555 (JP)
(72) Inventor: KOBAYASHI, Ryoma, Chiba-shi, Chiba 263-8555 (JP); TAKAKUSAGI, Yoichi, Chiba-shi, Chiba 263-8555 (JP)
(74) Representative: Winter, Brandl - Partnerschaft mbB
(86) International application number: PCT/JP2024/015508
(87) International publication number: WO 2024/232234

(57) **Abstract**

Provided is a transmission device capable of transmitting a substance to be administered while maintaining hyperpolarization ability of the substance to be administered with a simple configuration.

A transmission device 4 of the present invention is provided between a polarization device 3 and an imaging device 2 using nuclear magnetic resonance, and is used when a substance having been hyperpolarized by the polarization device 3 is transferred to a place of use of the substance. The transmission device 4 includes a transmission tube 41 that constitutes a transmission path through which the substance is transmitted, a magnet 42 that generates a magnetic field in the transmission path, and a yoke 43 that has a relative magnetic permeability of 100 or more, and that is disposed constituting a magnetic circuit with the magnet 42.

## Description

### Technical Field

The present invention relates to a transmission device and a transmission method for transmitting a hyperpolarized substance to be administered, for example.

### Background Art

For the purpose of improving sensitivity in imaging using nuclear magnetic resonance, a method for performing imaging has been conventionally proposed in which a substance (substance to be administered) is brought into a hyperpolarized state by a hyperpolarization method using a polarization device, and the substance to be administered that has become the hyperpolarized state (has hyperpolarization ability) is administered in vivo.

The polarization device and an imaging device (NMR device or MRI device) are each internally under a strong magnetic field environment of several tesla, so that the hyperpolarization ability of the substance to be administered can be maintained to some extent during processing performed in the polarization device and the imaging device. Unfortunately, the hyperpolarization ability of the substance to be administered is rapidly lost after the substance to be administered passes through a low magnetic field region. In general, the substance to be administered is administered in vivo on a side close to the imaging device, so that a path (transmission path) for transmitting the substance to be administered that has the hyperpolarization ability from the polarization device to the imaging device needs to be under a strong magnetic field environment.

For this problem, a method is considered in which a permanent magnet is disposed around the transmission path to generate a magnetic field in the transmission path. As an example of a transmission device in which permanent magnets are disposed, a transmission device using a magnet facing structure has been proposed (Non-Patent Literature 1) in which magnets are disposed facing each other on both sides of the path. Unfortunately, the magnet facing structure has a large leakage magnetic field, thus causing a problem that the leakage magnetic field becomes an obstacle to the imaging device. The magnet facing structure has also a problem in terms of safety, in which magnetic metal such as iron is attracted to the leakage magnetic field.

Additionally, a transmission device using a simple Halbach circuit structure has been proposed as a method for generating a stronger magnetic field in a transmission path (Non-Patent Literature 2). Unfortunately, the transmission device has a problem that structure of the transmission device becomes complicated to increase manufacturing cost of the transmission device because permanent magnets need to be disposed without a gap to obtain effect of the simple Halbach circuit structure, thus requiring not only a magnet having a special shape, but also an installation structure to be made more complicated and robust due to an increase in weight of a part including the magnets.

### Citation List

### Non Patent Literature

Non Patent Literature 1: Hyperpolarized water through dissolution dynamic nuclear polarization with UV-generated radicals COMMUNICATIONS CHEMISTRY (2020)3:57 Arthur C. Pinon et al.
Non Patent Literature 2: A magnetic tunnel to shelter hyperpolarized fluid REVIEW OF SCIENTIFIC INSTRUMENTS 86, 024101(2015)Jonas Milani et al.

### Summary of Invention

### Technical Problem

In view of the above problems, it is an object of the present invention to provide a transmission device and a transmission method capable of performing transmission while maintaining hyperpolarization ability of a substance to be administered with a simple configuration.

### Solution to Problem

The present invention provides a transmission device and a transmission method, the transmission device being used for transferring a substance having been hyperpolarized to a place of use of the substance, and the transmission device including: a tubular member that constitutes a transmission path through which the substance is transmitted; a magnet that generates a magnetic field in the transmission path; and a yoke member that has a relative magnetic permeability of 100 or more, and that is disposed constituting a magnetic circuit with the magnet.

### Advantageous Effects of Invention

The present invention enables providing a transmission device and a transmission method capable of transmitting while maintaining the hyperpolarization ability of a substance to be administered with a simple configuration.

### Brief Description of Drawings

FIG. 1 is a schematic explanatory diagram illustrating a general configuration of an imaging system.
FIG. 2 is an end view illustrating a configuration of a transmission device of the present invention.
FIG. 3 is a schematic explanatory diagram for illustrating a flow of magnetic lines of force in the transmission device of the present invention.
FIG. 4 is a lateral end view illustrating the configuration of the transmission device of the present invention.
FIG. 5 is an end view illustrating an example of a configuration of a transmission device according to a modified embodiment.
FIG. 6 is an end view illustrating another example of the configuration of the transmission device according to the modified embodiment.
FIG. 7 is an end view illustrating yet another example of the configuration of the transmission device according to the modified embodiment.
FIG. 8A is an end view illustrating yet another example of the configuration of the transmission device according to the modified embodiment.
FIG. 8B is an end view illustrating yet another example of the configuration of the transmission device according to the modified embodiment.
FIG. 9 is a side view illustrating another example of the configuration of the transmission device according to the modified embodiment.
FIG. 10 is a side view illustrating yet another example of the configuration of the transmission device according to the modified embodiment.
FIG. 11A is a plan view illustrating another example of the configuration of the transmission device according to the modified embodiment.
FIG. 11B is a plan view illustrating yet another example of the configuration of the transmission device according to the modified embodiment.
FIG. 12 is an explanatory diagram illustrating another example of the configuration of the transmission device according to the modified embodiment.
FIG. 13 is an explanatory diagram illustrating yet another example of the configuration of the transmission device according to the modified embodiment. Description of Embodiments

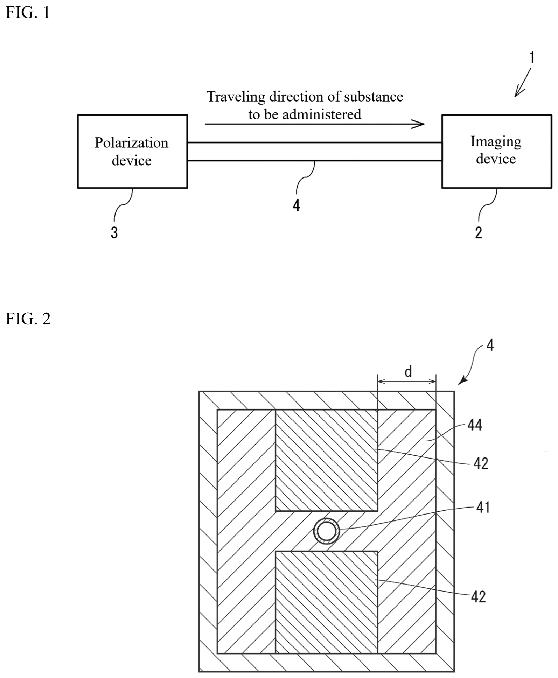

Hereinafter, an embodiment of the present invention will be described with reference to the drawings. FIG. 1 is a schematic explanatory diagram illustrating a general configuration of an imaging system 1 according to a first embodiment of the present invention. The imaging system 1 includes an imaging device 2, a polarization device 3, and a transmission device 4.

The imaging device 2 is configured to convert information inside a subject in vivo to be imaged into an image. For example, the imaging device 2 is a nuclear magnetic resonance (NMR) device or a magnetic resonance imaging (MRI) device using an NMR phenomenon.

The NMR device is configured to analyze a molecular structure by irradiating an object to be imaged disposed in a static magnetic field with a high-frequency signal, detecting a minute high-frequency signal (nuclear magnetic resonance signal: NMR signal) emitted from a sample, and extracting molecular structure information included in the nuclear magnetic resonance signal. The MRI device used in a medical field is configured to capture a tomographic image of an object to be imaged in real time using a magnetic resonance effect. That is, the imaging device 2 may function also as a detection device.

The imaging device 2 uses a method to capture an image in vivo to be imaged for the purpose of improving sensitivity in imaging, the method being performed by bringing a liquid substance to be administered into a hyperpolarized state (hyperpolarized) by hyperpolarization, and administering the substance to be administered having become the hyperpolarized state (has hyperpolarization ability) is into the in vivo to be imaged.

The polarization device (hyperpolarization device) 3 is configured to bring a substance to be administered into a hyperpolarized state by the hyperpolarization. The hyperpolarization refers to a technique for amplifying a nuclear magnetic resonance signal several 10 times to several tens of thousands times, and can enhance sensitivity of the imaging device 2 that performs imaging using nuclear magnetic resonance. Types of the hyperpolarization include a rare gas polarization method in which a substance to be administered is irradiated with a laser to polarize nuclear spin using a rare gas such as 3He or 129Xe, a para-hydrogen polarization method in which para-hydrogen is added by a chemical reaction to magnetize a target nucleus, and a dynamic nuclear polarization (DNP) method using double resonance of electron spin (free radical) and nuclear spin.

For example, when the imaging device 2 is an MRI device, the imaging device 2 uses a method for performing metabolic imaging by inducing a dynamic nuclear polarization phenomenon with respect to a ¹³C-labeled compound (reagent) in the polarization device 3, administering the ¹³C-labeled compound hyperpolarized in vivo as a contrast medium, observing a dynamic state of the ¹³C compound.

The substance to be administered having hyperpolarization ability is a substance having a polarization rate of 0.002% or more, and is a liquid reagent. Here, the polarization rate is a ratio of a direction of spin of a molecule in a substance. The polarization rate is expressed as 100% when the spin is aligned either upward or downward in all molecules in the substance, and 0% when the spin is aligned equally upward and downward in all molecules in the substance. When used in the imaging system 1, the polarization rate of the substance to be administered is preferably 1% or more, and more preferably 10% or more.

The transmission device 4 is internally provided with a transmission path for transmitting the substance to be administered and is configured to transmit the substance to be administered having hyperpolarization ability from the polarization device 3 to the imaging device 2 or near the imaging device 2 (a place where the substance to be administered is administered in vivo). The imaging device 2 and the polarization device 3 are each a device (strong magnetic field devices) that generate a strong magnetic field (from 3tesla), and are disposed apart from each other to be prevented from being affected by a mutual magnetic field (leakage magnetic field). For example, a position (safety line) having a magnetic field intensity of 5 gauss or less in the leakage magnetic field is said to be safe. Although a distance from each device to the safety line varies depending on a degree of the leakage magnetic field, the imaging device 2 and the polarization device 3 are disposed at least 1 m or more apart from each other, and may be disposed at an interval of 2 m or more, or may be disposed at an interval of 3 m or more. The imaging device 2 and the polarization device 3 may be disposed in different rooms in a medical institution or the like, and it is also assumed that the imaging device 2 and the polarization device 3 are disposed at an interval of 10 m to 100 m or more.

The imaging system 1 configured as described above can image the inside of a subject in vivo to be imaged by quickly transmitting the substance to be administered having hyperpolarization ability from the polarization device 3 to the imaging device 2 using the transmission device 4, and administering the substance to be administered into the subject in vivo to be imaged. That is, the polarization device 3 is a transfer source of the transmission path (transmission device 4), and the imaging device (detection device) 2 using a magnetic resonance method is a transfer destination of the transmission path (transmission device 4). The transfer destination of the transmission path (transmission device 4) can also be referred to as a place of use of the substance to be administered having hyperpolarization ability. As the place of use of the substance to be administered having hyperpolarization ability, other utilization methods are also conceivable in addition to the imaging device (detection device) 2 using the magnetic resonance method.

### <Problems of prior art>

As described above, when the substance to be administered is transmitted from the polarization device 3 to the imaging device 2, the hyperpolarization ability of the substance to be administered is preferably maintained as much as possible. The hyperpolarization ability of the substance to be administered can be maintained to some extent in a high magnetic field environment, but rapidly decreases in a low magnetic field environment. That is, the hyperpolarization ability of the substance to be administered is lost when the substance to be administered passes through a low magnetic field region, thus causing decrease in final signal intensity (decrease in sensitivity in imaging).

In view of such a problem, it is conceivable to configure a transmission device (transmission device with a strong magnetic field) having a transmission path (magnet tunnel) of a strong magnetic field for maintaining the hyperpolarization ability of the substance to be administered. For example, a transmission device using a simple Halbach circuit structure has been proposed to configure a transmission device with a strong magnetic field. Unfortunately, the simple Halbach circuit has various problems as follows.

First, four magnets need to be provided around the transmission path to configure the simple Halbach circuit. That is, the simple Halbach circuit needs to be provided with at least four magnets per section. The simple Halbach circuit also needs to dispose permanent magnets without any gap, and thus needs to use a magnet having a special shape because a magnet having a general shape for general use generates a gap. For this reason, the transmission device using the simple Halbach circuit has a problem that cost per magnet increases and the required number of magnets increases to increase manufacturing cost for forming the transmission path with a strong magnetic field.

The simple Halbach circuit is also increased in the number of magnets as described above to increase weight of the magnets and a size of the magnets, thus causing the transmission device to have a heavy configuration as a whole. Thus, a strong structure for supporting the heavy configuration is required. That is, the transmission device using the simple Halbach circuit requires an installation structure complicated and robust due to increase in weight of the transmission path, thus causing a problem that manufacturing cost of the transmission device increases. Additionally, a large and strong structure has a problem of low portability and lack of versatility.

Safety of a device having a magnet depends on a degree of a leakage magnetic field, and thus when the magnet itself generates a large leakage magnetic field, a magnetic body such as iron is attracted to the leakage magnetic field, which is very dangerous. While the transmission device using the simple Halbach circuit can generate a strong magnetic field, a leakage magnetic field cannot be completely eliminated, and thus there is still room for improvement from the viewpoint of safety.

As described above, the transmission device using the simple Halbach circuit has problems in terms of increase in manufacturing cost for configuring a transmission path with a strong magnetic field, increase in manufacturing cost due to complication and robustness of the installation structure, and safety. To solve these problems, the present inventors have devised a novel configuration of a transmission device to be described below, thereby solving the problems.

### <Configuration of transmission device of the present invention>

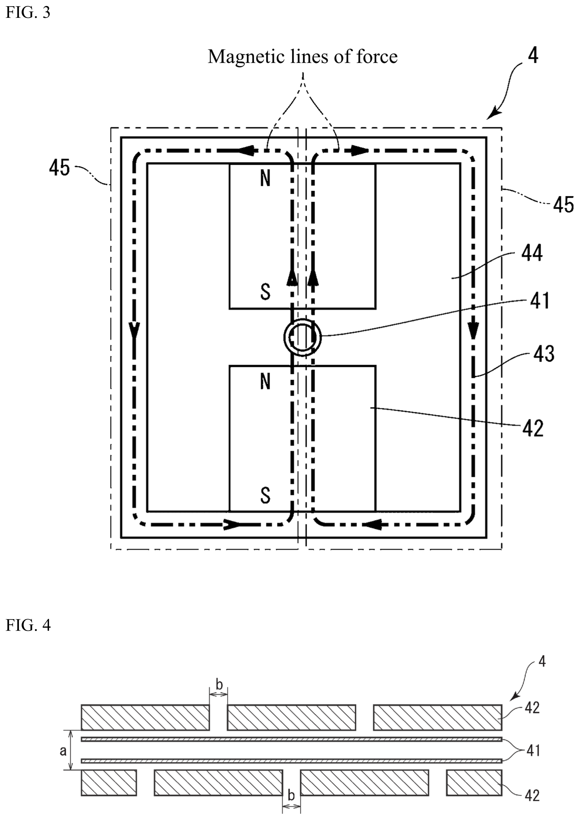

FIG. 2 is a sectional view illustrating a configuration of the transmission device 4 of the present invention. FIG. 3 is a schematic explanatory diagram for illustrating a flow of magnetic lines of force in the transmission device 4 of the present invention. FIG. 4 is a lateral sectional view illustrating the configuration of the transmission device 4 of the present invention. FIG. 4 does not illustrate a support 44 for convenience of illustration.

As illustrated in FIGS. 2 and 3, the transmission device 4 includes a transmission tube 41, a magnet 42, a yoke 43, and the support 44. The transmission device 4 is configured to linearly extend in a horizontal direction in the present embodiment, and each direction is defined as follows: a front side (side close to the polarization device 3) in an axial direction of the transmission device 4 when the imaging device 2 is viewed from the polarization device 3 (referred to below also simply as an "axial direction") is defined as a front surface side; a back side in the axial direction (side close to the imaging device 2) is defined as a back surface side, and the right as viewed from the front surface side to the back surface side is defined as a right side (the left as viewed therefrom is defined as a left side). The above is a description for convenience of defining each direction, and the transmission device 4 may not necessarily be linear or horizontal.

The transmission tube 41 is a member in a tubular shape (tubular member) having a circular section or a rectangular section. The transmission tube 41 has an internal space serving as a transmission path through which a substance to be administered is transmitted. That is, the transmission tube 41 can also be said to be a path forming member that forms the transmission path. The transmission tube 41 is made of a low magnetic permeability material. The low magnetic permeability material has a relative magnetic permeability (µ/µ₀) of 1.1 or less. Magnetic permeability in vacuum is represented by µ₀. For example, metal (copper, aluminum, or the like), resin, rubber, or the like having a relative magnetic permeability of 1.1 or less can be used as a material for forming the transmission tube 41. The material forming the transmission tube 41 is preferably a flexible material to secure flexibility of the transmission path.

The magnet 42 is a permanent magnet or an electromagnet. As the permanent magnet, a neodymium magnet, a samarium-cobalt magnet, a ferrite magnet, or the like can be used. The electromagnet is an instrument that generates a magnetic field by applying a current to a coil formed of a conductive wire. As the electromagnet, an electromagnet using a normal conducting wire, a superconducting magnet using a superconducting wire, or the like can be used. In the present embodiment, the magnet 42 is a prismatic or cylindrical permanent magnet.

The magnet 42 is disposed to generate a magnetic field in the transmission path (the internal space of the transmission tube 41), that is, to allow magnetic lines of force generated from the magnet 42 to pass through the transmission path. For example, an extension line of an imaginary line connecting an N-pole and an S-pole is disposed to pass through the transmission path.

One or more magnets 42 are provided. In the present embodiment, two magnets 42 are provided while being vertically disposed side by side in a certain section. One of the magnets 42 is disposed above the transmission tube 41, the other is disposed below the transmission tube 41. The magnets 42 are disposed with respective surfaces facing each other across the transmission tube 41 and having different polarities. That is, the two magnets 42 are disposed across the transmission tube 41 in a vertical direction in a certain section. One of the magnets 42 has the N-pole close to the transmission tube 41, and the other of the magnets 42 has the S-pole close to the transmission tube 41.

Alternatively, the two magnets 42 may be disposed laterally side by side in a certain section instead of the above-described configuration. This placement enables the two magnets 42 to be disposed across the transmission tube 41 in a left-right direction.

Here, a distance between the upper and lower magnets 42, that is, an inter-magnet distance "a" in the same section (see FIG. 4) is preferably as small as possible from the viewpoint of increasing magnetic field intensity of the transmission path. For example, the inter-magnet distance "a" in the same section can be 10 mm or less, and is preferably 8 mm or less, more preferably 5 mm or less, still more preferably 3 mm or less. Then, 2 mm or less is preferable.

Although the magnet 42 is not particularly limited in size, it has a size of 10 mm in the vertical direction (thickness from the N-pole to the S-pole), a size of 10 mm in the left-right direction, and a size of 50 mm in a front-back direction, for example. Here, the transmission device 4 has a length in the front-back direction (the length of the magnet 42 in the front-back direction) that is substantially equal to a separation distance between the imaging device 2 and the polarization device 3. As described above, the separation distance between the imaging device 2 and the polarization device 3 is at least 1 m or more, so that multiple magnets 42 need to be disposed side by side in the axial direction of the transmission path as illustrated in FIG. 4. Here, as for a distance between the magnets 42 adjacent to each other, that is, an inter-magnet distance "b" in the front-back direction (the axial direction of the transmission path), the inter-magnet distance "b" as small as possible enables increasing the magnetic field intensity of the transmission path. For example, the inter-magnet distance "b" in the axial direction can be 10 mm or less, and is preferably 8 mm or less, more preferably 5 mm or less, still more preferably 3 mm or less. Then, 2 mm or less is preferable.

When multiple magnets 42 are disposed side by side in the axial direction, the magnets 42 facing each other across the transmission tube 41 are preferably disposed while being displaced in the axial direction (having positions different in the axial direction) as illustrated in FIG. 4 rather than when positions (positions in the axial direction) of the magnets 42 facing each other across the transmission tube 41 are aligned. This placement allows the magnets 42 to be attracted and stabilized, and thus enables reducing the inter-magnet distance "b" in the axial direction (e.g., about 2 mm).

Returning to FIGS. 2 and 3, the yoke 43 is made of a material (high magnetic permeability material) having higher magnetic permeability than the low magnetic permeability material. The high magnetic permeability material has a relative magnetic permeability of 100 or more. For example, a general magnetic metal material, such as soft iron or pure iron, can be used as the material for forming the yoke 43.

The yoke 43 is provided forming a magnetic circuit 45 in a loop shape in which magnetic lines of force generated from the magnet 42 pass through the transmission path and connect the N-pole and the S-pole of the magnet 42. That is, the yoke 43 functions as a magnetic circuit forming member.

Specifically, the yoke 43 is provided in a loop shape connecting the N-pole and the S-pole of the magnet 42. The yoke 43 may be formed of one or more members each in a plate shape. For example, the yoke 43 can be formed of one member, or can be formed by appropriately combining a member in a flat plate shape, a member with an L-shaped section (L-shaped plate), a member in a U-shape turned sideways or U-shape, and the like.

When multiple magnets 42 are provided, the number of magnets 42 is not particularly limited, and each of the multiple magnets 42 is disposed on the magnetic circuit 45 with the N-pole and the S-pole in the same alignment (same direction of each of the magnets 42).

In the present embodiment, the yoke 43 is formed surrounding the magnet 42 and the transmission tube 41. Specifically, the yoke 43 of the present embodiment is integrally formed in a rectangular tube shape having a rectangular section, and the yoke 43 has inner surfaces in contact with an upper surface of the magnet 42 disposed above and a lower surface of the magnet 42 disposed below. The yoke 43 of the present embodiment is provided covering the outside of the magnet 42 and the transmission tube 41 without a gap. Thus, the magnetic lines of force generated by the magnet 42 come out upward from the N-pole of the magnet 42 disposed above, and then split into right and left along an upper wall of the yoke 43 as illustrated in FIG. 3. Each of the magnetic lines of force having split into right and left draws a U-shape along the shape of the yoke 43 in the order of the upper wall, a side wall, and a lower wall of the yoke 43 made of the high magnetic permeability material, and enters the S-pole of the magnet 42 disposed below. That is, a loop is formed by a straight line connecting the N-pole of the magnet 42 disposed above and the S-pole of the magnet 42 disposed below, and an outer part of the yoke 43 as viewed from the straight line. This loop serves as the magnetic circuit 45, and the yoke 43 exists without interruption between the N-pole of the magnet 42 disposed above and the S-pole of the magnet 42 disposed below. Thus, a leakage magnetic field can be reduced. Although the magnetic circuit 45 is expressed as a loop because it is described in section, the magnet 42 and the yoke 43 actually extend in the axial direction. Thus, the magnetic circuit 45 also has a tubular shape in which the loop is three-dimensionally extended in the axial direction. When a through-hole is formed in the side wall of the yoke 43, the magnetic circuit 45 has a shape bypassing the through-hole (surrounding the outside of the through-hole).

A shortest distance "d" (see FIG. 2) between the side wall of the yoke 43 and the magnet 42 is larger than at least the inter-magnet distance "a" in the same section. Consequently, magnetic lines of force exiting the N-pole of the magnet 42 disposed below and traveling toward the S-pole of the magnet 42 disposed above can be prevented from flowing to the side wall of yoke 43. That is, the magnetic field intensity (magnetic flux density) of the transmission path can be prevented from decreasing.

The support 44 is made of a low magnetic permeability material, and is interposed between the transmission tube 41, the magnet 42, and the yoke 43 to maintain a positional relationship among the transmission tube 41, the magnet 42, and the yoke 43 (supports each member). In the present embodiment, the support 44 is provided among the transmission tube 41, the magnet 42, and the yoke 43 without a gap. As a material forming the support 44, resin, wood, or the like can be used, for example.

The transmission device 4 of the present invention with the above configuration enables the transmission path (the internal space of the transmission tube 41) to have a magnetic flux density of 10 mT (tesla) or more (a magnetic field environment of 10 mT or more). Here, the magnetic flux density in the transmission path is in a part where the magnetic flux density is minimum in the transmission path. The magnetic flux density below is meant similarly. For example, when the magnet 42 having a thickness of 10 mm from the N-pole to the S-pole is disposed above and below the transmission path (transmission tube 41) as in the present embodiment (FIG. 2), the transmission path can be set in a magnetic field environment of 350 mT or more. Besides this, when the magnets 42 facing each other across the transmission tube 41 are disposed while being displaced in the axial direction as illustrated in FIG. 4, the transmission path can be set in a magnetic field environment of 440 mT or more.

As described above, the present invention enables at least the transmission path to be under a magnetic field environment of 10 mT or more to maintain a strong magnetic field by providing the yoke 43 for forming the magnetic circuit 45 in a loop shape connecting the N-pole and the S-pole of the magnet 42. This configuration enables transmitting a substance to be administered while maintaining hyperpolarization ability of the substance to be administered. Consequently, the substance to be administered can be transmitted to the imaging device 2 while performance of the substance to be administered hyperpolarized by the polarization device 3 is maintained, so that a degree of freedom in installation of the polarization device 3 and the imaging device 2 can be increased. That is, the polarization device 3 and the imaging device 2 are each installed in a separate room, and the polarization device 3 and the imaging device 2 are connected to each other by the transmission device 4, for example. Then, the substance to be administered having been hyperpolarized by the polarization device 3 is put into the transmission path and transmitted to the imaging device 2 disposed away from the polarization device 3 while being hyperpolarized, and the substance to be administered having been hyperpolarized is administered in vivo and imaged with high imaging sensitivity by the imaging device 2, and thus a clear captured image can be obtained.

The yoke 43 can be made of a general magnetic metal material such as soft iron, and thus is smaller and cheaper than a magnet. Consequently, manufacturing cost for forming the transmission path with a high magnetic field can be reduced, and complication and robustness of the installation structure can be avoided. That is, the substance to be administered can be transmitted with a simple configuration while the hyperpolarization ability of the substance to be administered is maintained. In particular, when the polarization device 3 and the imaging device 2 need to be installed away from each other due to convenience of facilities, a large cost reduction effect can be obtained.

Additionally, a path through which magnetic force flows can be controlled using the yoke 43 made of a magnetic metal material, so that a strong magnetic field can be formed inside the transmission tube 41. Thus, a magnetic field generated from the magnet 42 can be efficiently applied to the substance to be administered in the transmission tube 41.

The multiple magnets 42 are provided on the magnetic circuit 45 to enable further increase of the magnetic field (magnetic flux density) in the transmission path according to the present invention. Thus, the hyperpolarization ability of the substance to be administered can be effectively maintained.

The yoke 43 includes one or more members each in a plate shape according to the present invention. Thus, the yoke 43 can be formed by combining members each having a highly versatile shape, that is, inexpensive materials, so that manufacturing cost for forming a transmission path with a high magnetic field can be reduced.

The yoke 43 is also formed surrounding (covering) the magnet 42 and the transmission tube 41 according to the present invention. Thus, the yoke 43 exists without interruption between the N-pole of the magnet 42 disposed above and the S-pole of the magnet 42 disposed below, so that a leakage magnetic field can be reduced to further increase the magnetic field (magnetic flux density) in the transmission path.

### <Configuration of transmission device of modification>

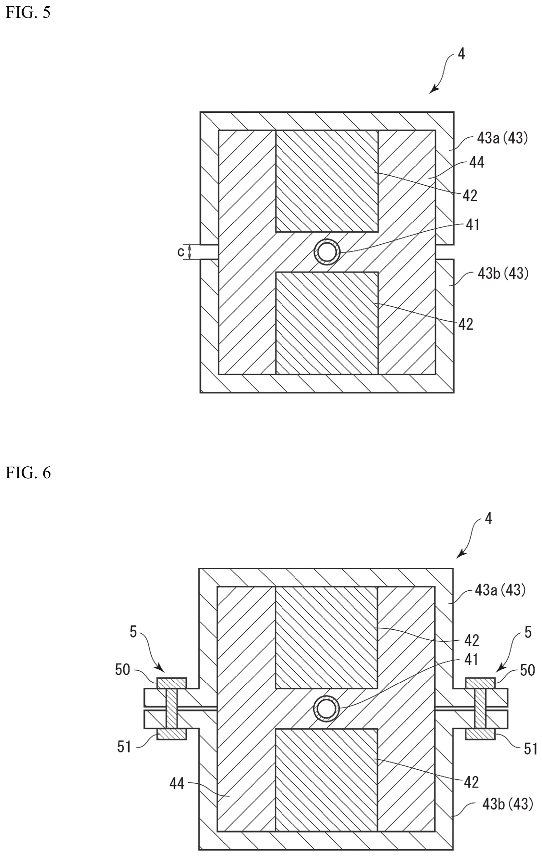

FIG. 5 is an end view illustrating an example of a configuration of a transmission device 4 according to a modified embodiment. As illustrated in FIG. 5, a yoke 43 may be divided into multiple members (a first member 43a and a second member 43b) by partially changing the configuration of the above-described embodiment. In this configuration, the multiple members (the first member 43a and the second member 43b) may be in contact with each other or may be separated from each other. FIG. 5 illustrates the example in which the first member 43a and the second member 43b are provided away from each other by a predetermined separation distance "c" in the vertical direction. When magnetic lines of force pass between the first member 43a and the second member 43b, the magnetic lines of force spread in accordance with a length of the separation distance "c". The separation distance "c" is set to a distance that allows a magnetic circuit 45 to maintain at least a transmission path in a magnetic field environment of 10 mT or more even when the magnetic lines of force are spread. For example, the separation distance "c" can be set to a distance shorter than three times a thickness of the yoke 43, for example, and is preferably set to a distance shorter than twice the thickness of the yoke 43. Then, a distance shorter than the thickness of the yoke 43 is preferable. Thus, even the configuration as illustrated in FIG. 5 enables forming a magnetic circuit having a loop shape in section similar to the magnetic circuit 45 as illustrated in FIG. 3, so that the same effects as those of the above embodiment can be obtained.

FIG. 6 is an end view illustrating another example of the configuration of the transmission device 4 according to the modified embodiment. As illustrated in FIG. 6, a yoke 43 may be divided into multiple members (a first member 43a and a second member 43b), and then the multiple members (the first member 43a and the second member 43b) may be fixed by a fixing member 5. For example, extended parts (flange parts) extending outward from respective opposing parts of the first member 43a and the second member 43b may be formed, and the extended parts of the first member 43a and the second member 43b may be fixed to each other by a fixing member 5. The fixing member 5 is made of a high magnetic permeability material having a relative magnetic permeability equivalent to that of the yoke 43 (the same material as that of the yoke 43), for example. The fixing member 5 includes a bolt 51 and a nut 50, for example, but is not particularly limited. Even the configuration as illustrated in FIG. 6 enables forming a magnetic circuit having a loop shape in section similar to the magnetic circuit 45 as illustrated in FIG. 3, so that the same effects as those of the above embodiment can be obtained. Additionally, a degree of freedom of the shape of the yoke 43 increases, and thus manufacturing cost of the yoke 43 can be reduced.

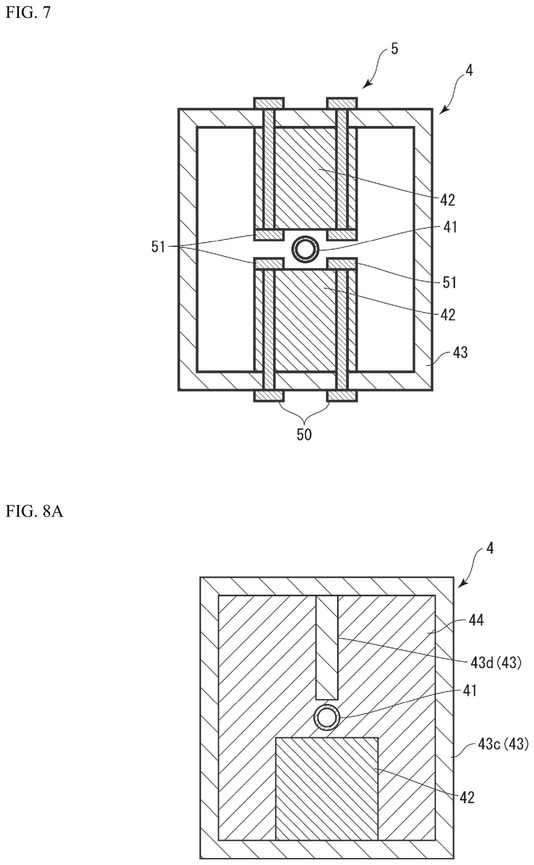

FIG. 7 is an end view illustrating yet another example of the configuration of the transmission device 4 according to the modified embodiment. As illustrated in FIG. 7, a yoke 43 and a magnet 42 may be directly fixed by a fixing member 5. This configuration enables eliminating a support 44, so that manufacturing cost for forming a transmission path with a high magnetic field can be reduced. Then, when the fixing member 5 includes a bolt 51 and a nut 50, and the yoke 43 and the magnet 42 are fixed by the bolt 51 and the nut 50, the bolt 51 and the nut 50 are preferably disposed such that the bolt 51 has an axis in a direction coinciding or substantially coinciding with a direction connecting a S-pole of the magnet 42. Even the configuration as illustrated in FIG. 7 enables forming a magnetic circuit having a loop shape in section similar to the magnetic circuit 45 as illustrated in FIG. 3, so that the same effects as those of the above embodiment can be obtained.

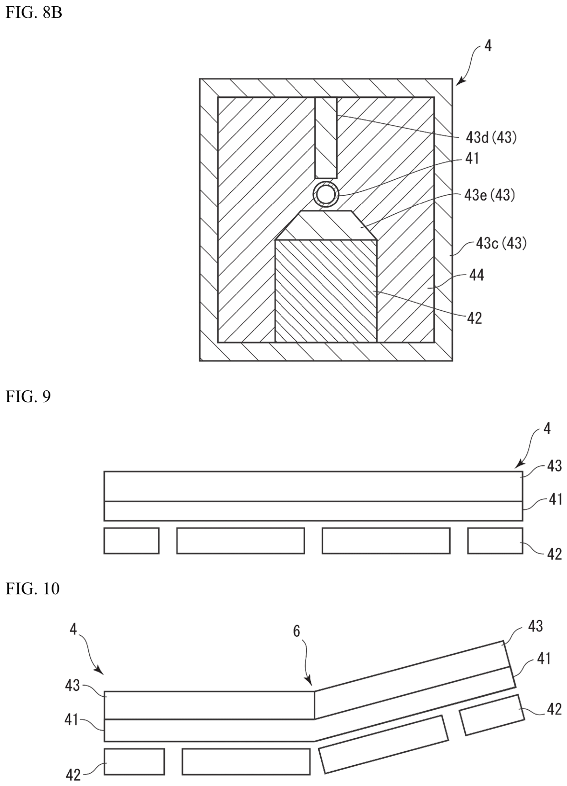

FIG. 8A is an explanatory diagram illustrating yet another example of the configuration of the transmission device 4 according to the modified embodiment. FIG. 8B is an explanatory diagram illustrating yet another example of the configuration of the transmission device 4 according to the modified embodiment. As illustrated in FIGS. 8A and 8B, a magnet 42 may be disposed on only one of upper, lower, left, and right sides when viewed from a transmission tube 41. This configuration may include not only a main yoke 43c formed surrounding the magnet 42 and the transmission tube 41, but also a first auxiliary yoke 43d that is dense and extends from a side close to the transmission tube 41 to the main yoke 43c, the side being opposite to the magnet 42 as viewed from the transmission tube 41. Consequently, magnetic lines of force generated by magnet 42 exit upward from an N-pole of the magnet 42 to pass through a transmission path (the transmission tube 41), then pass through the inside of the first auxiliary yoke 43d to reach the main yoke 43c. Thus, the magnetic lines of force can be efficiently guided to the main yoke 43c, so that a magnetic circuit 45 can be efficiently formed to maintain the transmission path at a strong magnetic field. As illustrated in FIG. 8B, not only a main yoke 43c and a first auxiliary yoke 43d, but also a second auxiliary yoke 43e that is dense and interposed between a transmission tube 41 and a magnet 42, can be provided. The second auxiliary yoke 43e has a shape (conical shape or pyramidal shape) with a sectional area decreasing toward a central axis of the transmission tube 41 from the magnet 42 toward the transmission tube 41. Consequently, the magnetic lines of force generated by the magnet 42 can be concentrated toward the center of the transmission tube 41 along the shape of the second auxiliary yoke 43e, so that the transmission path can be maintained at a stronger magnetic field. As described above, even the configuration as illustrated in each of FIGS. 8A and 8B enables forming a magnetic circuit having a loop shape in section similar to the magnetic circuit 45 as illustrated in FIG. 3, so that the same effects as those of the above embodiment can be obtained. Additionally, the number of magnets 42 can be reduced, so that manufacturing cost for forming a transmission path with a high magnetic field can be reduced.

FIG. 9 is a side view illustrating yet another example of the configuration of the transmission device 4 according to the modified embodiment. FIG. 10 is a side view illustrating an example of a configuration of a transmission device 4 of an embodiment using the embodiment of FIG. 9. FIGS. 9 and 10 do not illustrate a support 44 for convenience of illustration. When magnets 42 are disposed on only one of the upper, lower, left, and right sides when viewed from a transmission tube 41 as illustrated in FIG. 9, a changing part 6 for changing a traveling direction of a transmission path can be provided in the transmission device 4 as illustrated in FIG. 10. The changing part 6 illustrated in FIG. 10 bends the traveling direction of the transmission path in the vertical direction (direction in which an S-pole and an N-pole are aligned). At this time, the magnets 42 are disposed outside the bend, and a yoke 43 is disposed inside the bend. The yoke 43 has a shape following the bend of the transmission path. That is, a magnetic circuit 45 is formed following the traveling direction of the transmission path. Here, when placement of the magnets 42 is adjusted to locate a gap between the corresponding magnets 42 at the changing part 6 (bent part), general-purpose columnar magnets can be used as the magnets 42 without using magnets each in a special shape. Meanwhile, although the yoke 43 needs to have a shape suitable for the bend, the yoke 43 is made of a general metal material such as soft iron and thus is processed more easily than the magnet 42. Consequently, increase in manufacturing cost can be suppressed to a minimum requirement, and a degree of freedom of the transmission path can be increased.

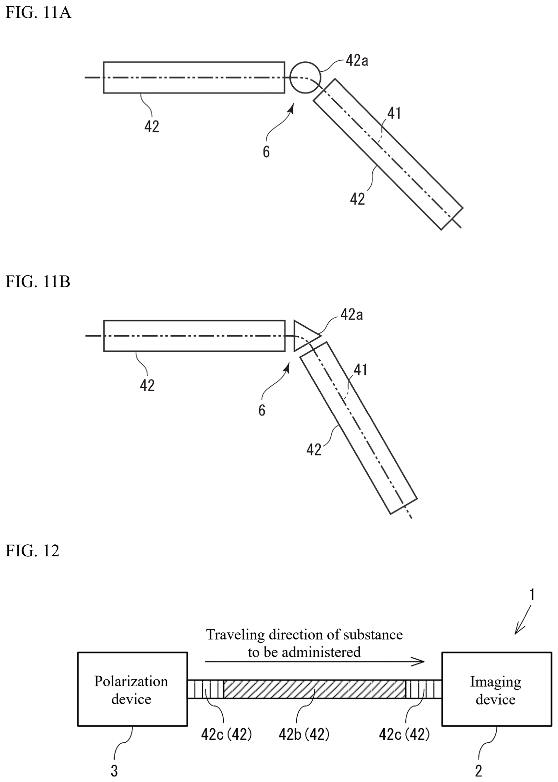

FIGS. 11A and 11B are each a plan view illustrating yet another example of the configuration of the transmission device 4 according to the modified embodiment. FIGS. 11A and 11B do not illustrate a yoke 43 and a support 44 for convenience of illustration. As illustrated in FIGS. 11A and 11B, the transmission device 4 can be provided with a changing part 6 that bends a traveling direction of a transmission path in the horizontal direction (direction perpendicular to a direction in which an S-pole and an N-pole are aligned). For example, the changing part 6 can be formed by disposing a magnet for a relay (relay magnet) 42a between magnets 42 each in a columnar shape. For example, a relay magnet 42a (columnar shape) with a cross section in a circular shape as illustrated in FIG. 11A or a relay magnet 42a (prismatic shape) with a cross section in a polygonal shape as illustrated in FIG. 11B can be used. Consequently, increase in manufacturing cost can be suppressed to a minimum requirement, and a degree of freedom of the transmission path can be increased. In particular, when the relay magnet 42a in a columnar shape is used, a distance between the relay magnet 42a and each of the magnets 42 on both sides of the relay magnet 42a can be made constant regardless of a bending angle. Thus, the bending angle can be freely set. Although FIG. 11B illustrates an example of a cross section in a triangular shape, the cross section may have a fan shape or any one of various polygonal shapes.

FIG. 12 is an explanatory diagram illustrating yet another example of the configuration of the transmission device 4 according to the modified embodiment. As illustrated in FIG. 12, magnets 42 can include an electromagnet 42c as a magnet 42 at least at an end of a transmission path in an axial direction. As described above, the imaging device 2 and the polarization device 3 are each a device with a strong magnetic field. Thus, when only permanent magnets are used as the magnets 42, both ends of the transmission device 4 in its longitudinal direction may not be brought close to the imaging device 2 and the polarization device 3. Using only the permanent magnets causes a large gap (zero magnetic field) to be generated between the transmission device 4 and each of the imaging device 2 and the polarization device 3, so that hyperpolarization ability of a substance to be administered is lost when passing through the gap. In contrast, when the magnet 42 at the end of the transmission path in the axial direction is composed of the electromagnet 42c, a current is allowed to flow to generate a magnetic force in the electromagnet 42c only during transmission of the substance to be administered, and the current is not allowed to flow to prevent the magnetic force from being generated in the electromagnet 42c when the substance to be administered is not transmitted. Consequently, when the substance to be administered is not transmitted, the magnetic force is not generated at both end parts of the transmission device 4 in the longitudinal direction. Thus, the transmission device 4 can be brought close to the imaging device 2 and the polarization device 3, so that the hyperpolarization ability of the substance to be administered can be effectively maintained, and safety can be enhanced.

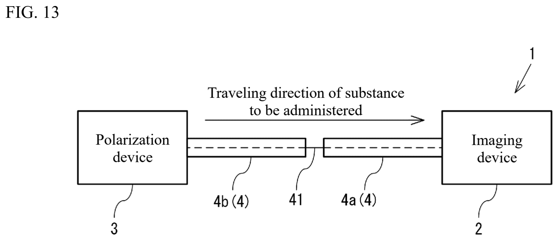

FIG. 13 is an explanatory diagram illustrating yet another example of the configuration of the transmission device 4 according to the modified embodiment. As illustrated in FIG. 13, a configuration other than a transmission tube 41 (a magnet 42, a yoke 43, a support 44) may be divided into a first transmission device 4a and a second transmission device 4b in the middle of a traveling direction. This configuration allows the first transmission device 4a and the second transmission device 4b to be disposed apart from each other by a predetermined distance. Then, a gap between the first transmission device 4a and the second transmission device 4b serves as a low magnetic field (zero magnetic field) region, so that a separation distance between the first transmission device 4a and the second transmission device can be set to a distance at which passing time of a substance to be administered is within one second. Consequently, decrease in hyperpolarization ability of the substance to be administered can be minimized.

The transmission device of the present invention corresponds to the transmission device 4 of the above embodiment. Similarly, the imaging device corresponds to the imaging device 2, the polarization device corresponds to the polarization device 3, the tubular member corresponds to the transmission tube 41, the magnet corresponds to the magnet 42, the magnetic circuit forming member corresponds to the yoke 43, the fixing member corresponds to the fixing member 5, and the changing part corresponds to the changing part 6. Besides this, the present invention is not limited to the present embodiment, and various other embodiments can be applied. The specific configurations and the like described in the above-described embodiments are merely examples, and can be appropriately changed according to an actual product.

For example, the yoke 43 in the above-described embodiments may include outer protrusions protruding outward from the upper wall, the side wall, and the lower wall. This configuration allows the outer protrusions to less affect the magnetic circuit 45, so that the same effect as that of the above embodiments can be obtained.

Although the support 44 is provided among the transmission tube 41, the magnet 42, and the yoke 43 without a gap in the above-described embodiments, the present invention is not necessarily limited thereto. The support 44 does not contribute to a configuration of the magnetic circuit, and thus may have any shape as long as a function of supporting each member can be exhibited. For example, the support 44 may be provided only in a part of an internal space of the yoke 43, or the support 44 may include a part in a shape protruding outward from the yoke 43.

### Industrial Applicability

The present invention is available for an industry in which information inside a subject in vivo to be imaged is converted into an image using an imaging device using nuclear magnetic resonance.

### Reference Signs List

- 1: imaging system
- 2: imaging device
- 3: polarization device
- 4: transmission device
- 41: transmission tube
- 42: magnet
- 43: yoke
- 44: support

## Claims

1. A transmission device that is used for transferring a substance having been hyperpolarized to a place of use of the substance, the transmission device comprising:
a tubular member that constitutes a transmission path through which the substance is transmitted;
a magnet that generates a magnetic field in the transmission path; and
a yoke member that has a relative magnetic permeability of 100 or more, and that is disposed constituting a magnetic circuit with the magnet.

2. The transmission device according to claim 1, wherein
the magnet constitutes multiple magnets in the magnetic circuit, and
the yoke member forms the magnetic circuit in a loop shape in which magnetic lines of force generated from the magnet pass through the transmission path and connect an N-pole and an S-pole of the magnet.

3. The transmission device according to claim 1 or 2, wherein the yoke member includes one or more members each in a plate shape.

4. The transmission device according to any one of claims 1 to 3, further comprising:
a fixing member that fixes the magnet and the yoke member and has a relative magnetic permeability of 100 or more.

5. The transmission device according to any one of claims 1 to 4, wherein the yoke member is formed surrounding the magnet and the tubular member.

6. The transmission device according to any one of claims 1 to 5, further comprising:
a changing part that changes a traveling direction of the transmission path and causes the magnetic circuit to follow the traveling direction of the transmission path.

7. The transmission device according to any one of claims 1 to 6, wherein at least the magnet at an end of the transmission path in an axial direction is composed of an electromagnet.

8. The transmission device according to any one of claims 1 to 6, wherein at least the magnetic field applied to the transmission path has a magnetic flux density of 10 mT or more.

9. The transmission device according to any one of claims 1 to 6, wherein at least the magnetic field applied to the transmission path has a magnetic flux density of 100 mT or more.

10. The transmission device according to any one of claims 1 to 6, wherein the transmission path includes at least a transfer source serving as a hyperpolarization device.

11. The transmission device according to any one of claims 1 to 6, wherein the transmission path includes at least a transfer destination serving as a detection device or an imaging device using a magnetic resonance method.

12. A transmission method comprising:
transferring a substance having been hyperpolarized to a place of use of the substance using a transmission device by putting the substance into a transmission path provided in the transmission device that includes a tubular member that constitutes the transmission path through which the substance is transmitted, a magnet that generates a magnetic field in the transmission path, and a yoke member that has a relative magnetic permeability of 100 or more, and that is disposed constituting a magnetic circuit with the magnet.
